# EUROPEAN PATENT SPECIFICATION

(11) **EP 3 283 143 B1**
(45) Date of publication and mention of the grant of the patent: **22.01.2020**
(21) Application number: 16740467.2
(22) Date of filing: 22.01.2016
(51) Int. Cl.: A61M 5/14, F16M 11/20, F16M 11/04, F16M 11/24, F16M 11/28, F16M 11/42

(54) **STAND FOR IV BAGS AND A SYSTEM FOR STORING MULTIPLE IV STANDS**
STÄNDER FÜR IV-BEUTEL UND SYSTEM ZUR AUFBEWAHRUNG VON MEHREREN IV-STÄNDERN
SUPPORT POUR POCHES IV ET SYSTÈME POUR STOCKER DES SUPPORTS IV MULTIPLES

(30) Priority: 23.01.2015 SE 1500037
(43) Date of publication of application: 21.02.2018
(73) Proprietor: Tarsus Products AB, 891 50 Örnsköldsvik (SE)
(72) Inventor: SJÖBLOM, Olow, 891 50 Örnsköldsvik (SE)
(86) International application number: PCT/SE2016/000002
(87) International publication number: WO 2016/118061

(56) References cited:
- DE-U1- 9 201 579
- SE-A1- 1 200 026
- SE-A1- 1 200 026
- US-A- 4 744 536
- US-A- 4 807 837
- US-A- 4 892 279
- US-A1- 2005 269 464
- US-B1- 8 567 730

## Description

### Field of the Invention

The present invention concerns a stand for IV bags (infusion bags) in accordance with the claims.

### Background of the Invention

In health care, a large number of intravenous infusions containing, for example, drips, blood and the like are given daily. These infusions are usually given by fluids in so-called IV bags (infusion bags) being transferred to the bloodstream of the patient via an IV line or the like. The transfer of fluid (liquid) from the IV bag to the patient is usually done by the infusion bag being hung up on a so-called infusion stand (IV stand) at a higher vertical level than the patient, allowing for the liquid to be transferred to the patient by the force of gravity. Alternatively, the liquid in the IV bag may be transferred to the patient through pumping by an infusion pump or similar.

The IV stand is typically comprised of a vertical pole (post), a suspension device for the bags and a base stand (foot), leg stand or the like. To satisfy the need of being able to adjust the height of the IV bag relative to the patient, the vertical length of the pole is telescopically adjustable. A problem with existing types of IV stands is that the locking devices that lock the stand's mutually telescopic parts may be difficult to quickly and reliably operate between a locked and unlocked position and vice versa. There is thus a need for an improved variant of a locking device for the pole's parts with which a simpler adjustment of the height of the IV stand may occur.

One problem with existing types of IV stands is that they usually take up considerable space both in use and when being stored when not in use. This problem is especially troublesome in conjunction with the storage of IV stands when they are not being used. A problem with existing IV stands is that they are usually designed to stand on the floor or the like when not in use. This means that the storage of multiple IV stands typically becomes disordered with stands easily hooking into each other, and that storage of IV stands takes considerable space, especially if they are stored standing on the floor. The disordered manner in which IV stands are stored even causes workplace related problems.

In order to reduce the amount of space required to store IV stands, the IV stands' base stand (foot) has been made collapsible. This has led to IV stands taking up relatively less space but the storage of IV stands is still of a disorderly character. There is therefore a need for a substantially improved IV stand which takes little space and which with simplicity may be stored together in large numbers simultaneously. The problem with most of the existing types of IV stands is that they are not suitable to be hung up in a collapsible (retracted) position.

A further problem with existing types of base supporting devices (such as a collapsible base stand (foot), collapsible leg stand or the like) for example for IV stands, is that they may be difficult and blockish to operate between a retracted position and an extended position and vice versa. Thus, it may be difficult for a user, especially for users with weak muscles, to extend and retract the leg stands of the known types of IV stands.

Further, there is also the problem that the leg stands, in a simple and easy way, may be extended and retracted at the same time that it reliably remains positioned in the collapsed position and the extended position. Especially when storing multiple IV stands simultaneously hung next to each other, there exists a problem if the base leg stands do not in a reliable manner stay in a collapsed position. With the known design of leg stands, there is a risk that the leg stands involuntarily open to an extended position and thereby complicate the storage of multiple IV stands stored hung up close together.

A problem with the above mentioned leg stands is that these leg stands must be both functionally reliable (regarding, for example the leg stand design), while also being cost-effective to manufacture. Known designs of base leg stands have the problem that they are comprised of a large number of parts and thus they become time consuming and/or expensive to manufacture and assemble. For example, the attachment of the wheels is time consuming.

A further problem with the known types of IV stands, especially collapsible types, is that they are often difficult to clean and because of this it may be difficult to maintain a high level of hygiene. For example, these stands have many nooks and crannies that may be difficult to clean and thereby bacteria and the like may propagate in these. There also exist problems when IV stands are to be sterilized by high temperatures such as for example in an autoclave. This requires that the choice of materials used for the IV stand is of such a type that it can tolerate (withstand) the high temperatures of an autoclave or similar. Problems exist with materials used for bearings and other components in the IV stand, that these materials are not able to tolerate high temperatures. During the sterilization of existing IV stands, there exist problems with oil or other lubricants in wheel bearings and similar being removed by high temperatures.

Another problem with existing IV stands, that are not collapsible, is that they are bulky and thereby difficult to clean in sterilization equipment. Further, existing IV stands are difficult to move back and forth from sterilization equipment and back and forth from storage without them coming into contact with the floor and thereby lose their sterilization. The ability to properly sterilize an IV stand, and other equipment, is especially important after they have been used in the treatment of patients with infectious diseases, bacterial diseases or similar such as Ebola.

### Prior Art

A large number of different types of stands for IV bags are previously known. For example SE536440 describes a variant of a stand for infusion bags which is comprised of two suspension members that at a first glance are similar to the suspension members in accordance with the present application. The suspension member in accordance with SE536440 however, differs significantly from the suspension member in accordance with the present patent application. For example, the design is comprised of a plurality of parts, substantially more than the present design is comprised of, which contributes to relatively higher manufacturing costs. For example, the design's base leg stand consists of a large number of parts. The base leg stand's design also means that great care must be maintained when cleaning the stand for it to maintain a high level of hygiene. For example, the design according to its description includes profiles of a type that may be difficult to keep clean from bacteria and the like.

Swedish patent application SE1200026 discloses a system for storing infusion bags. It also describes a variant of the IV stand in accordance with SE536440. The design in accordance with SE1200026 includes embodiments of a base leg stand which includes a plurality of interconnected plates in which the base stand's legs are pivotally arranged. The base stand's (leg stand's) design has the disadvantage that it is comprised of a plurality of components which means that it will be expensive to manufacture, both from the standpoint of components and the assembly. The base stand's (leg stand's) design also has the disadvantage that the base stand's (leg stand's) temporary fixation in the retracted and extended position can be improved. The design also has the earlier described disadvantage that the suspension member only can be used to hang a maximum of two IV bags in contrast to a variant of the present invention which allows for more than two to be hung up. Further the design includes nooks and crannies which calls for meticulous cleaning for the IV stand to maintain a high level of hygiene. For example, the design according to its description includes profiles of a type that may be difficult to keep clean from bacteria and the like.

### Purpose of the Invention

The main purpose of the present invention is to create an improved IV stand which solves or reduces at least one of the above mentioned technical problems. A further purpose of the present invention is to create an IV stand which may be retracted and stored in a space efficient manner. A yet further purpose of the present invention is to create an IV stand which may be cost-effectively manufactured and at the same time hold a high hygienic standard. A still further purpose of the present invention is to create an IV stand which includes an improved function for retraction and extension (fold in and fold out) of the stand.

### Brief Description of the Drawings

The present invention will in the following text be described in more detail with reference to the accompanying schematic drawings which by way of example show the currently preferred embodiment of the invention.

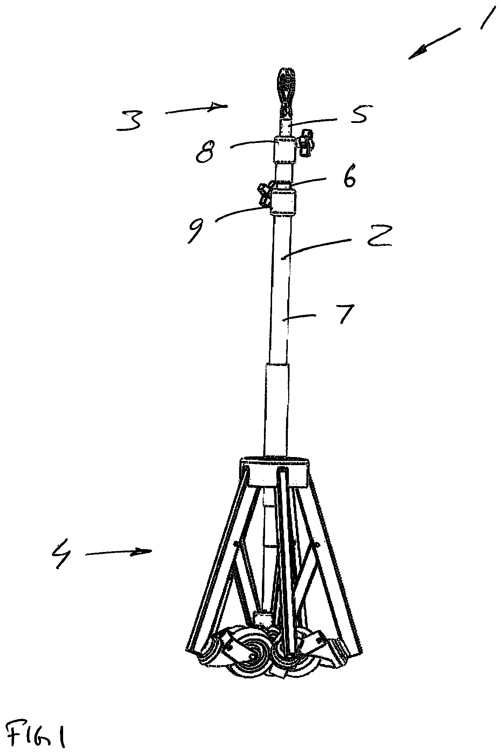

Fig. 1 shows a retracted IV stand in accordance with a first embodiment.
Fig. 2 shows an extended IV stand in accordance with a first embodiment.
Figs. 3A-3D show exemplifying locking devices for the poles parts.

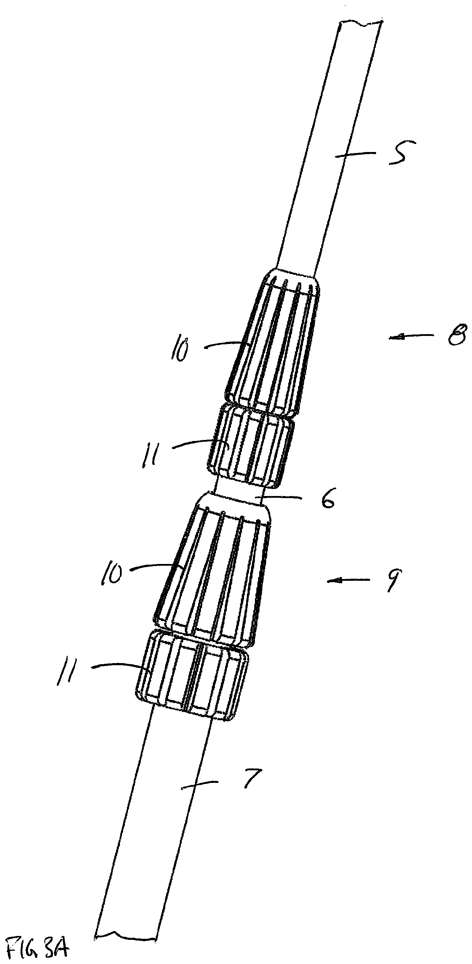

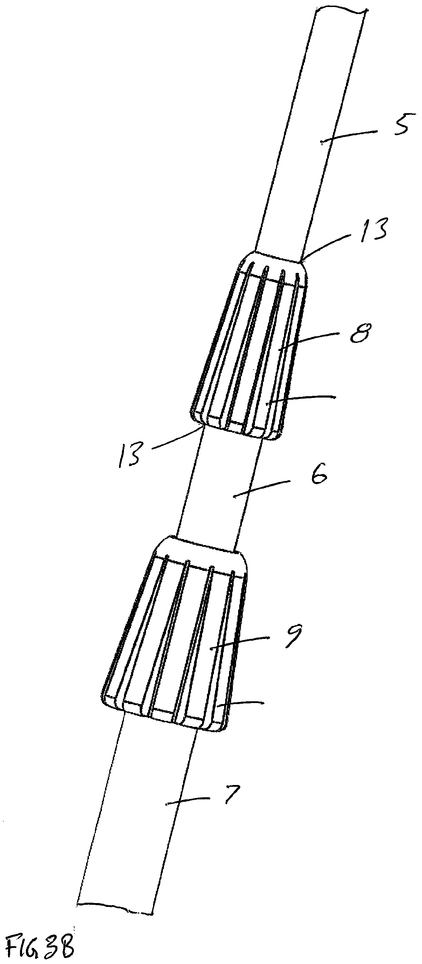

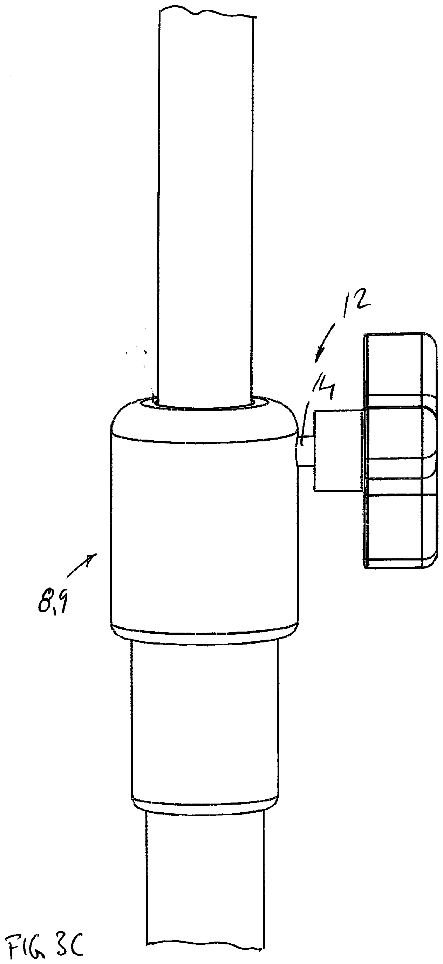

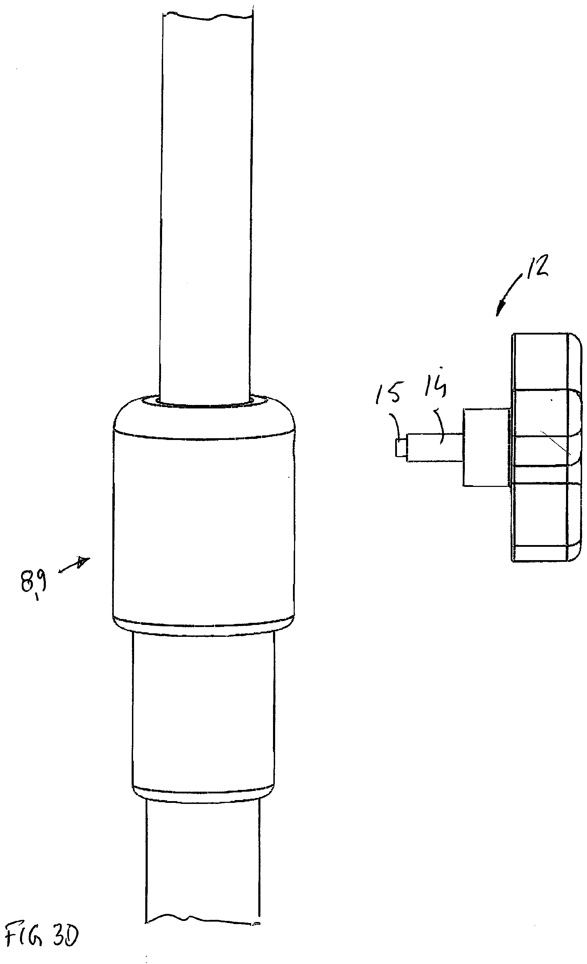

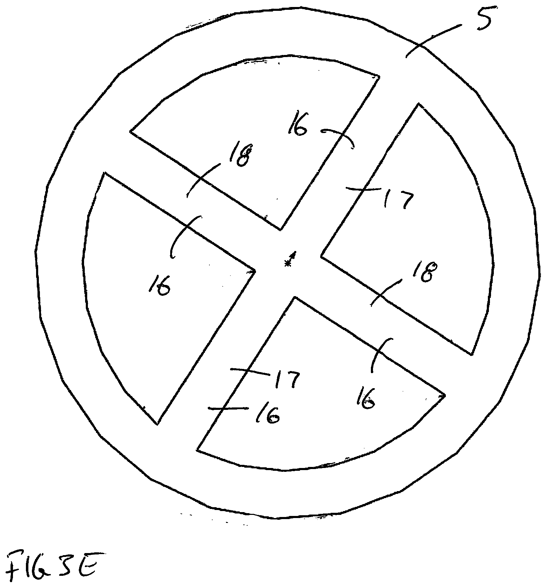

Fig. 3 shows an alternative embodiment where the pole part in a cross-section is comprised of at least one reinforcement.

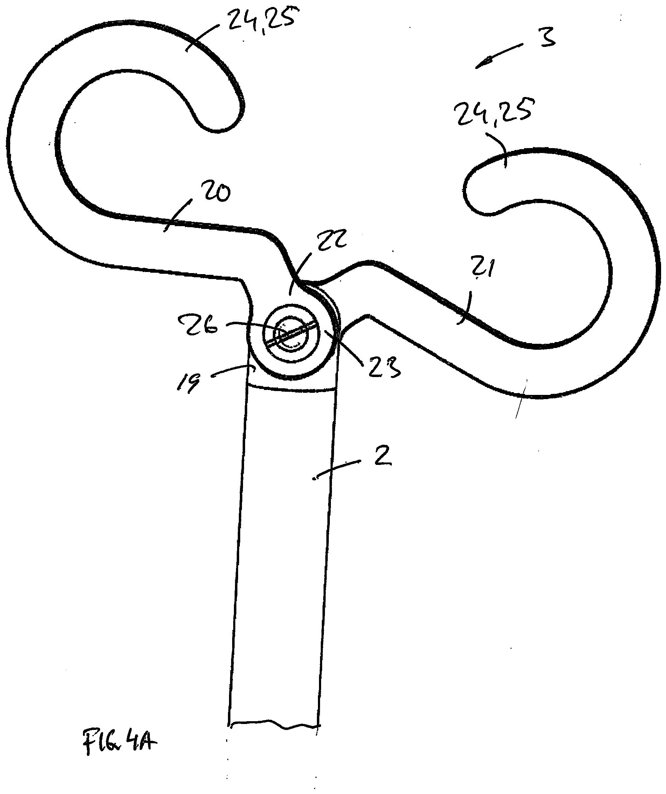

Fig. 4A shows a first embodiment of the present suspension device.

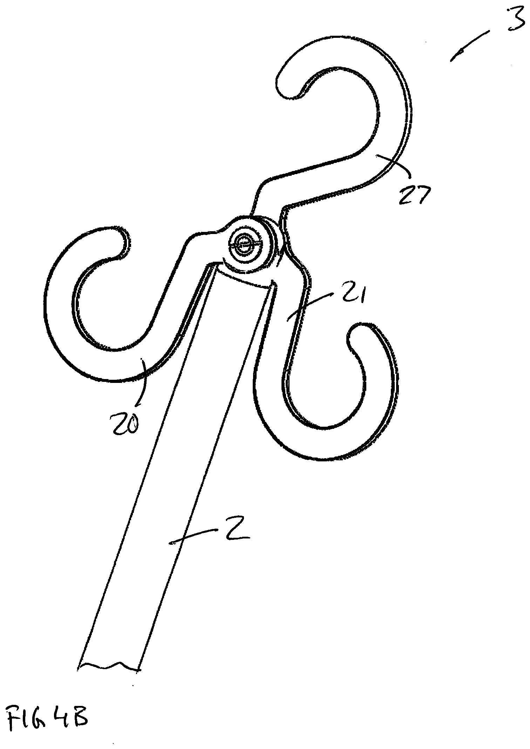

Fig. 4B shows a first alternative embodiment of the IV stand's suspension device.

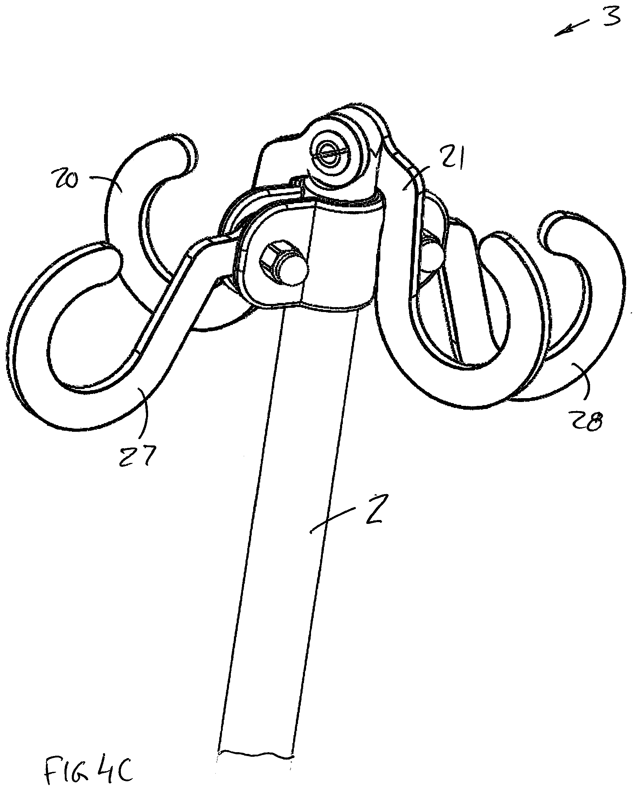

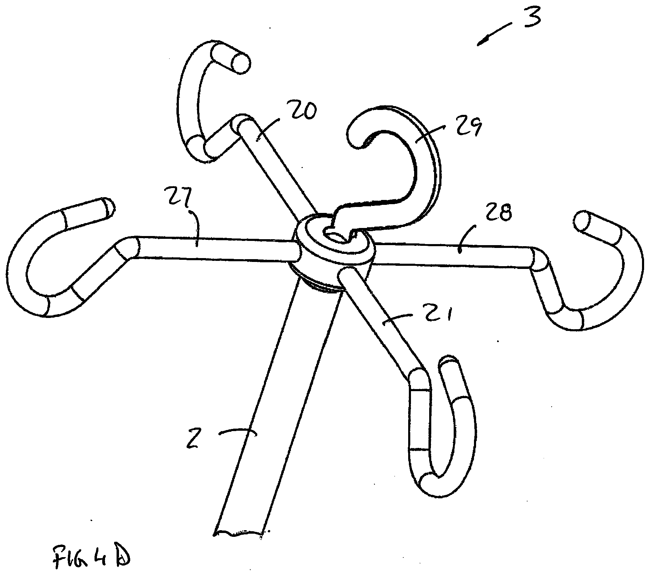

Figs. 4C and 4D show alternative embodiments of the IV stand's suspension device.

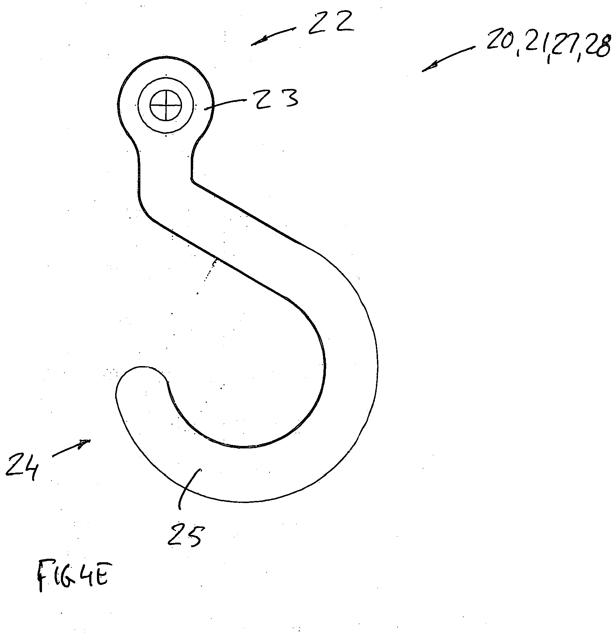

Fig. 4E shows a suspension device according to an exemplifying preferred embodiment.

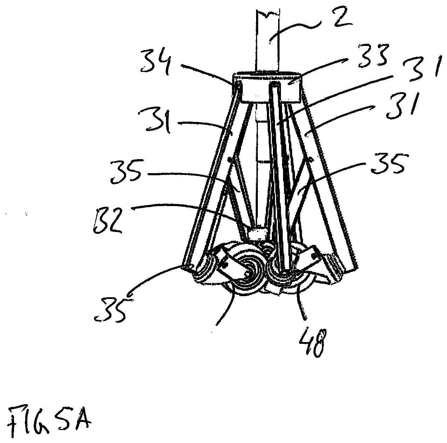

Fig. 5A shows the first part (lower hub, fixed hub) in more detail.

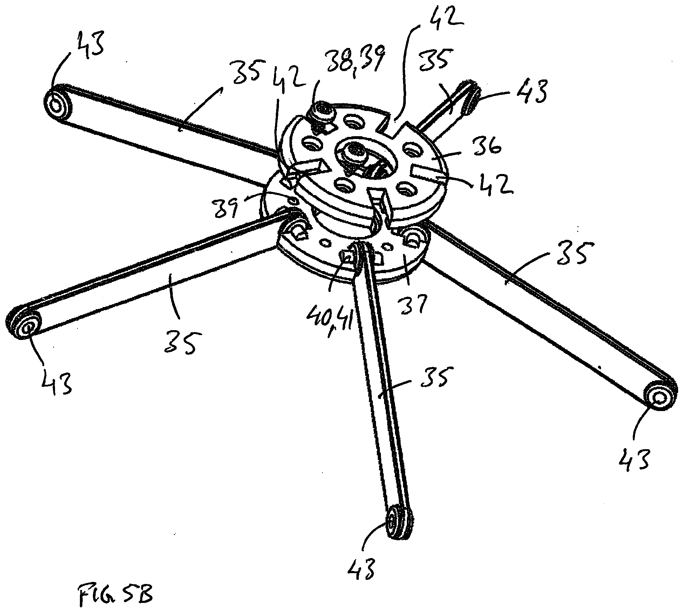

Fig. 5B shows the second part (upper hub, variable hub) in more detail.

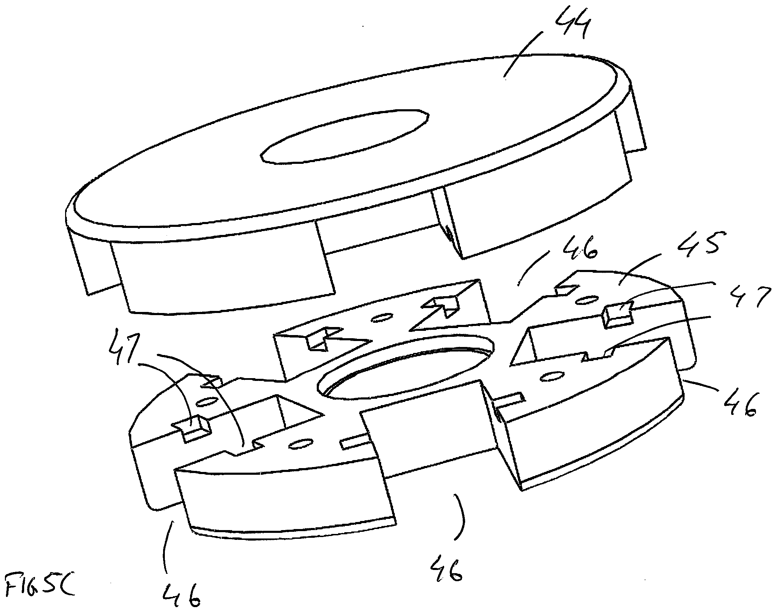

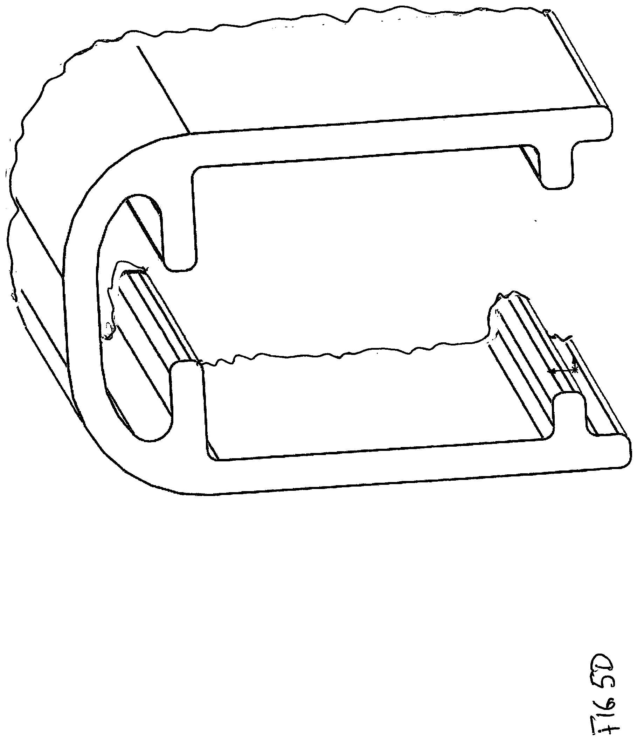

Figs. 5C and 5D show profiles included in the legs and a cross-section of the profiles.

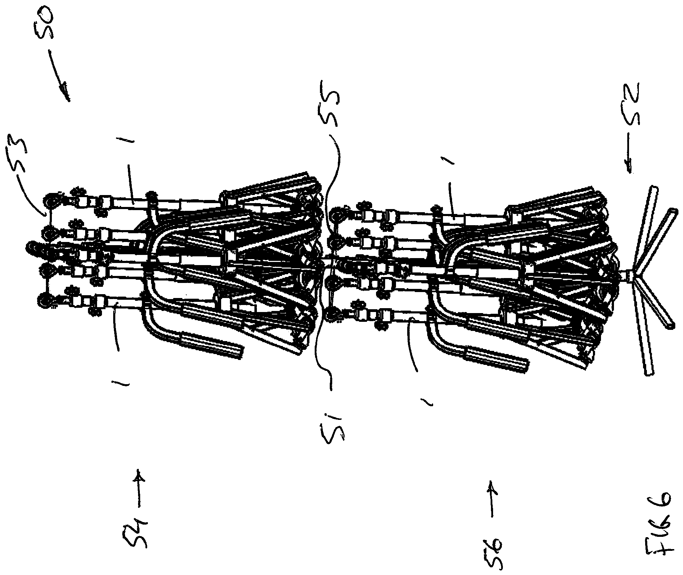

Fig. 6 shows a first exemplifying variant of a system with which the IV stand may be hung up (suspended).

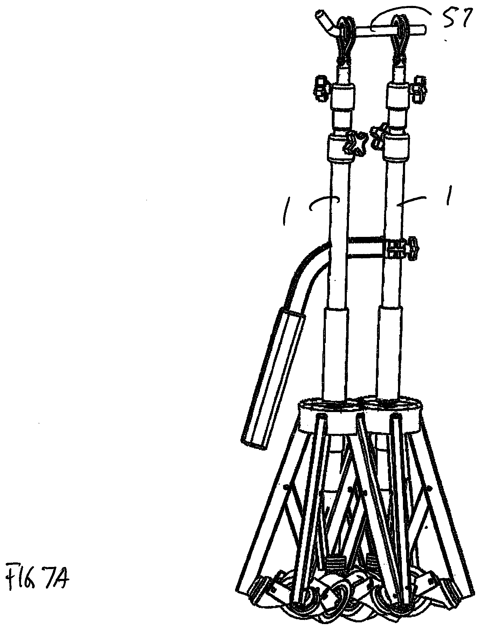

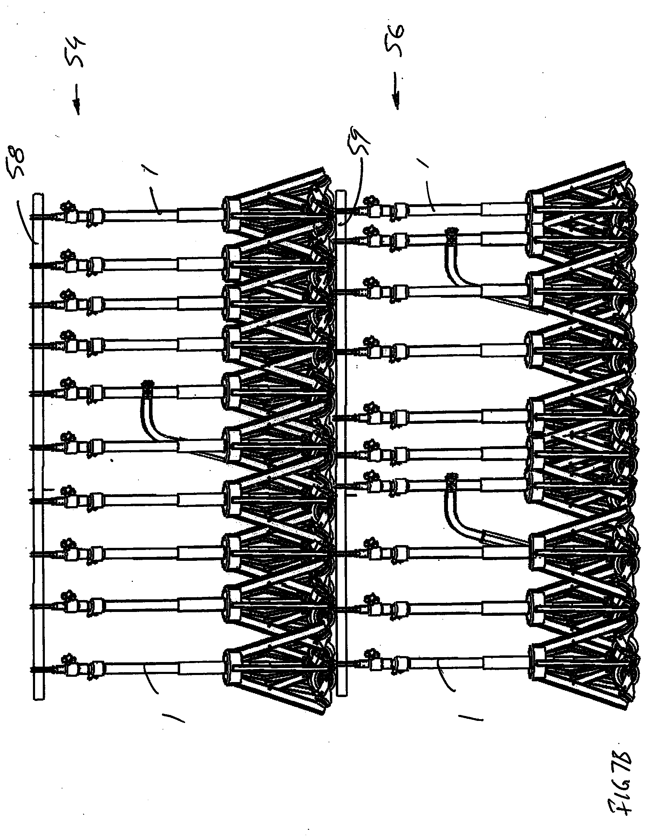

Figs. 7A and 7B show variants of a system with which the IV stand may be hung up.

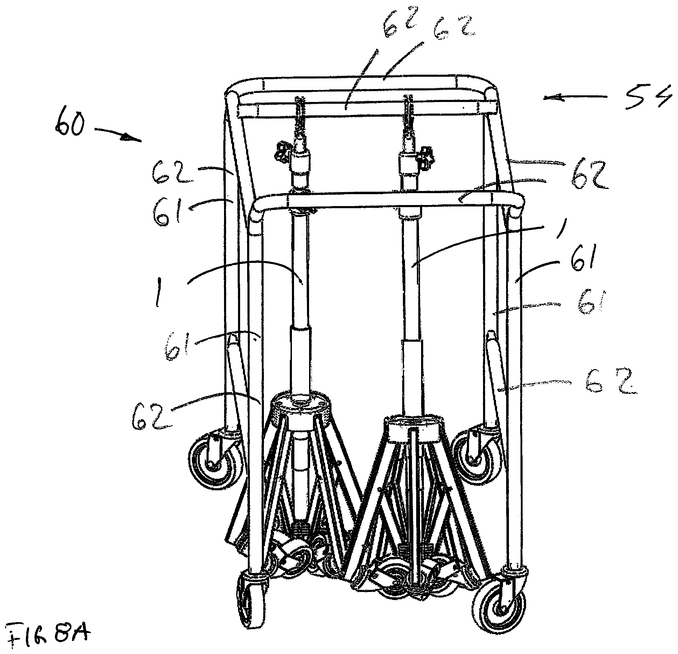

Figs. 8A to 8D show further exemplifying variants of the system.

### Detailed Description of the Invention

Referring to the figures, an exemplifying embodiment of an IV stand 1 in accordance with the present invention is shown. The term IV stand (infusion stand) 1 also refers to designs which are known as infusion (IV) carts, supports, IV poles (posts) and similar designs. The present invention in alternative embodiments also concerns variants of systems to store and/or move the present stand in an elective or determined number.

The IV stand 1 is suitable to be used for hanging up at least one infusion (IV) bag (not shown in the figures) on at least one vertical level. In preferred embodiments, the IV stand is suitable for hanging up at least two infusion (IV) bags on at least one first vertical level, or alternatively at least one first vertical level and at least one second vertical level. The IV stand 1 is comprised of at least one pole 2, at least one first suspension device 3 for at least one IV bag and at least one base supporting device 4 (foot, base stand, floor-support device).

A specific feature of the preferred embodiments of the IV stand 1 is that the suspension device 3 for hanging up IV bags also has a function as a suspension device for the IV stand 1 in its entirety, when the IV stand is not in use for infusions. For example the IV stand, when not in use, may be suspended (hung up) on a hanging stand or other hanging device such as a rack or another for the purpose suitable device for suspension of the stand.

In the figures, the shown preferred embodiment of the IV stand according to the invention, the suspension device 3 for infusion bags and the suspension device for the IV stand in its entirety are integrated into a suspension device with both functions. In alternative embodiments, the suspension device or suspension devices for hanging up IV bags and the suspension device for suspending the IV stand may instead be comprised of separate suspension devices.

The pole 2 is preferably made of a pipe, tube, profile or the like. Preferably, the pole's 2 length is adjustable. The length of the pole 2 may be adjusted by the pole being telescopically adjustable. The pole 2 is preferably made of a pipe (tube), profile or similar. Preferably, the pole's 2 length is adjustable. The length of the pole 2 may be adjusted by the pole being telescopically adjustable. The pole 2 is comprised at least one first part **5** and at least one second part 6 and preferably at least one third part 7 which are telescopically arranged in relation to each other. The first part 5 is insertable into the second part 6. Alternatively, the second part 6 may be insertable into the first part 5. The second part 6 is insertable into the third part 7. Alternatively, the third part 7 may be insertable into the second part 6. The first part 5 may be locked to the second part 6 with at least one first locking device **8** or the like. The second part 6 may be locked in relation to the third part 7 with at least one second locking device **9.** In alternative embodiments, the pole 2 may be comprised of more or fewer parts than in the exemplified three parts.

With reference to figures 3A and 3B is shown a part of the included pole where the included locking devices 8 and 9 are comprised of locking devices which include at least one first locking member **10** and at least one second locking member **11** which with a mutual rotation of the locking members in the one direction lock them together with each other and with a mutual rotation of the locking members in the opposite direction disengages (unlocks) the locking function.

With reference to figure 3C, an alternative variant of the locking devices 8 and 9 are shown where the locking engagement occurs with at least one screw joint **12** or similar. The screw joint 12 in the exemplifying embodiment includes at least one handle (knob), or another variant of a maneuvering device. Alternatively, another for the purpose suitable technology may be used for the locking devices.

In a preferred embodiment, the IV stand 1 includes at least one scraping function with which the entire or partial surface on at least one of the pole's parts is scraped off with a mutual movement of one pole part in relation to the other pole part. This function is achieved by at least one scraping device **13,** such as an edge, ring, bearing, or seal of flexible and elastic material which surrounds the pole's circumference. In the figure, exemplifying positions for the scraping device 13 are shown. The scraping device may be integrated with the locking device or consist of an attachment which is not integrated with the locking device. The scraping device 13 acts with a springy force against the pole's surface which allows for a scraping off of any contaminants on the surface to occur.

With reference to figure 3D, it is shown how the locking device includes a locking member comprised of a screw joint 12. Specific to the invention in accordance with the embodiment is that the screw joint's 12 screw **14** includes a tip **15** of a relatively softer material which reduces the risk of damage to the pole parts.

With reference to figure 3E, a cross-section of a profile, pipe (tube) or the like included in the pole 2 in accordance with the alternative embodiment is shown. Specific to the embodiment is that at least one of the pole's 2 profiles, pipes or similar includes at least one reinforcement (stiffening) **16.** The reinforcement 16 may be of different forms but in the exemplifying embodiment it includes at least one first intermediate wall **17** and at least one second intermediate wall **18** which cross one another and form a cross-shaped cross section. In alternative embodiments, the reinforcement's cross section may be of another for the purpose suitable form with more of fewer intermediate walls. By way of this design with at least one reinforcement, the pole part's 5 bending stiffness (rigidity) increases and the hardware thickness of the pole part may be reduced. The design in accordance with the alternative embodiment reduces the risk of the thinnest part bending or otherwise being damaged. A corresponding effect may be achieved by the pole part 5 lacking the reinforcement and it being made of thicker hardware.

At its top end the pole's 2 pole part 5 has or is attached to at least one bracket (fastener) **19** for the first suspension device, for an IV bag or IV bags. The bracket's 19 design (shape, form) may vary to a large extent within the scope of the present invention. It is for example conceivable that the first suspension device is detachably connected together with the pole via a quick coupler (fastener). It is also conceivable that the bracket 19 is permanently attached to the upper end of the pole.

With reference to figures 4A to 4D, variants of suspension devices for IV bags and even the suspension device for the IV stand in its entirety is shown in more detail. The suspension device 3 is comprised of at least one first suspension member **20** and preferably at least one second suspension member **21.**

In the exemplifying embodiment shown in figure 4A, the first suspension member 20 and the second suspension member 21 consist of hook-like designs (claw-like designs). The hook-like suspension members 20 and 21 have in their one end **22** an eyelet **23,** loophole or similar. The other end **24** of the hook-like suspension device consists of bent part (curved part) **25.** The bent part 25 is preferably radius-shaped. In alternative embodiments, the bent part 25 may be of another for the purpose suitable shape. The suspension member 20 and the suspension member 21 are pivotally attached to a bracket 19 on the pole 2 via an axle **26** in the pole's horizontal direction. The bracket 19 includes at least one through hole (not shown in the figures). The first suspension member 20 and the second suspension member 21 are attached to the bracket 19 with a screw joint, axle or similar.

Figure 4A shows the suspension member pivotally arranged to a common rotational axle 26 and that the suspension members 20 and 21 are maneuverable between at least one first position where the suspension members are suitable for holding up at least one IV bag and at least one second position where the suspension members are turned upward in the vertical direction which allows suspension of the stand 2 in a vertical position. This position coincides, or essentially coincides, with the vertical direction of gravitational pull. By way of this design the suspended IV stands do not collide with one another, they may instead be hung up essentially without hooking into other suspended IV stands.

With reference to figure 4B, a first alternative embodiment is shown where the IV stand includes at least one first suspension member 20 and at least one second suspension member 21. The first suspension member 20 and the second suspension member 21 are utilized in the extended position to suspend IV bags. In a retracted position they may be used to suspend the IV stand. The design in the exemplifying embodiment includes at least one third suspension member **27.** The third suspension member 27 is used for suspending the IV stand in its entirety. The third suspension member 27 in the exemplifying embodiment consists of a claw-like (hook-like) element. The third claw-like member 27 may be permanently arranged or pivotally arranged in relation to its attachment.

With reference to figure 4C, a second alternative embodiment of the IV stand's suspension device 3 is shown which includes at least one first suspension member 20, at least one second suspension member 21, at least one third suspension member 27 and at least one fourth suspension member **28.** In the exemplifying embodiment, the first suspension member 20 and the second suspension member 21 are positioned on a first vertical level and the third suspension member 27 and the fourth suspension member 28 are positioned on a second vertical level. The first suspension member 20, the second suspension member 21, the third suspension member 27 and the fourth suspension member 28 may be comprised of the suspension members shown in the figures. In alternative embodiments, they may consist of other for the purpose suitable suspension members.

With reference to figure 4D, a third alternative embodiment of the IV stand is shown which includes at least one first suspension member 20, at least one second suspension member 21, at least one third suspension member 27 and at least one fourth suspension member 28. In the exemplifying embodiment, the first suspension member 20 and the second suspension member 21 are positioned on a first vertical level and the third suspension member 27 and the fourth suspension member 28 are positioned on a second vertical level. The design in the exemplifying embodiment includes at least one fifth suspension member **29.** The design differs from the previous embodiments because the suspension members are not able to turn (rotate, pivot). In alternative embodiments it is however conceivable that they are able to turn (rotate, pivot).

With reference to figure 4E, a preferred embodiment of the claw-like suspension member is shown. The design according to figure 4E differs from the earlier disclosed variants of hooks by way of its opening being adapted to facilitate the suspension of the IV stand in its entirety.

With reference to figure 5A, the base supporting device 4 is shown in more detail. The base supporting device 4 in the exemplifying figures consists of a foot (base stand, floor-support device) **30.** The foot 30 is comprised of at least three legs **31** and preferably five legs 31. The foot 30 is further comprised of at least one, in relation to the pole, fixed part (hub) **32** and one, in relation to the pole, turning part (hub) **33.** The first part and the second part have in the exemplifying embodiment round cross-sections. In alternative embodiments, the first part and the second part may however have another for the purpose suitable cross-sectional shapes.

Each respective leg 31 is in its first end **34** articulated and pivotally fastened to the turning part 33. Each respective leg further includes at least one strut **35** which in its one end is attached to the leg 31. In its other end, the strut 35 is pivotally attached to the fixed part (hub) 32.

With reference to figure 5B, the fixed part is shown in more detail. The fixed part (hub) 32 is comprised of at least one first section **36** and at least one second section **37.** The first section 36 and the second section 37 are attached to each other or coupled together with each other with at least one attachment device **38.** The attachment device 38 may be comprised of attachment members **39** such as screw, threaded holes, quick fasteners or similar. The attachment of the struts 35 to the fixed part is accomplished via at least one axle **40** or similar which is positioned in a recess (space) **41** which is formed between the first section 36 and the second section 37. The strut 35 is via the axle 40 pivotally arranged relative to the fixed part 32. The fixed part 32 also includes at least one recess **42** for each respective strut 35 so they may rotate around the rotational axis. The axle 40 is preferably integrated with the strut 35. The strut 35 in the exemplifying embodiment includes an attachment socket (bracket) **43** for an axle or similar attachment device with which the strut 35 is pivotally attached to the leg 31.

Further, the fixed part 32 also preferably includes at least one bracket for the pole 2. The attachment between the pole 2 and the fixed part (hub) 32 may be accomplished by an attachment member, bracket element or similar suitable for the purpose. For example the pole 2 and the fixed part 32 may be attached to each other with the aid of screw joints or with other for the purpose suitable fasteners. Characteristic for the present base supporting device is that it, in difference to earlier variants of the IV stand, includes a first part and a second part which are attached to each other.

With reference to figure 5C, a variant of the turning part (hub) 33 is shown in more detail. The turning part (hub) 33 in the exemplifying embodiment is comprised of at least one first section **44** and at least one second section **45.** At least the lower section (the second section 45) of the turning part 33 has a corresponding number of recesses **46** as its number of legs. The legs are suitable for attachment to the turning hub via at least one axle (not shown in the figures) which is suitable for attachment to at least one groove **47** adjacent to the recesses 46. The groove 47 is preferably transversely directed in relation to the recesses 46 (preferably also transversely directed in relation to the hub's radial direction. The axle may be integrated with the leg 31 or consist of a separate part attached to the leg. The groove 47 in the preferred embodiment is also a bearing (pivot) for the axle (provided that the sections 44 and 45 are made of a material that allows a bearing [pivoting] effect between the axle and the first section and/or the second section).

The hub and the leg are preferably made of a material which provides a sufficient articulation (bearing, pivoting) between the leg and hub. By choosing the right material it is possible for the articulation to occur in the material which means that a separate bearing may be omitted from the design.

With reference to figure 5D is shown in a cross-section (part of the profile included in the leg) an exemplifying embodiment of the leg's profile. Specific to the leg's profile in the preferred embodiment is that it has an open form. The profile's open form results in that cleaning fluids/disinfection fluids do not in any substantial amount remain in it, but instead runs out from the profile after disinfection, cleaning or similar has been accomplished. In alternative embodiments, the profile's cross-section may be of another for the purpose suitable open form (other than a u-shape). The leg's profile may be made from aluminum, polymer material (such as a plastic material) or another for the purpose suitable material. The leg's profile preferably consists of a material which can tolerate temperatures, at least 100 degrees C, that are necessary for the sterilization of the profiles. The material should preferably be of a type which can tolerate its treatment in an autoclave. In variants of the IV stand that are not intended to be treated in an autoclave or other sterilization or cleaning treatments, the leg stands may be made of another for the purpose suitable material or material combinations.

The strut 35 is preferably made of a material which has an articulating (pivoting) effect. Further, the strut 35 is preferably made of a material which can tolerate temperatures that are necessary for the sterilization of it in for example an autoclave. For example, the material may consist of a polymer material.

Each respective leg 31 includes in its other end 35 or ends proximity at least one wheel **48.** The wheel's 48 design may vary to a significant extent within the scope of the present invention. The wheel 48 consists preferably of a wheel design which is rotatable around a vertical axle. In alternative embodiments, the wheels may be of another for the purpose suitable type of wheel design than those exemplified. In alternative embodiments, one or more of the wheels may include a locking function (not shown in the figures) which stops the wheel (wheels) from turning. Specific to the wheel design in accordance to the present invention is that the wheels include a variant of a quick coupler (fastener) with which the wheels (wheel modules) may be attached to the leg's profile. An exemplifying embodiment of this is shown in the figures. The wheel is preferably made of a material or material combinations which can tolerate the temperature that is necessary for the sterilization of it in for example an autoclave or other for the purpose suitable sterilization or cleaning device. In alternative embodiments, the wheels, where they are not exposed to high temperatures, may be of a material or material combinations that do not tolerate (or to a limited degree tolerate) high temperatures.

The vertical pole 2 is suitable for attachment in a fixed or detachable (removable) manner to the IV stand's base supporting device (foot). The movable part may be moved along the pole's length between a first (lower) position and a second (higher) position. The movable part is preferably articulated in relation to the pole. The articulation may consist of a for the purpose suitable articulation.

The hub and the leg are preferably made of a material which provides a sufficient articulation (bearing, pivoting) between the leg and hub. By choosing the right material it is possible for the articulation to occur in the material which means that a separate bearing may be omitted from the design.

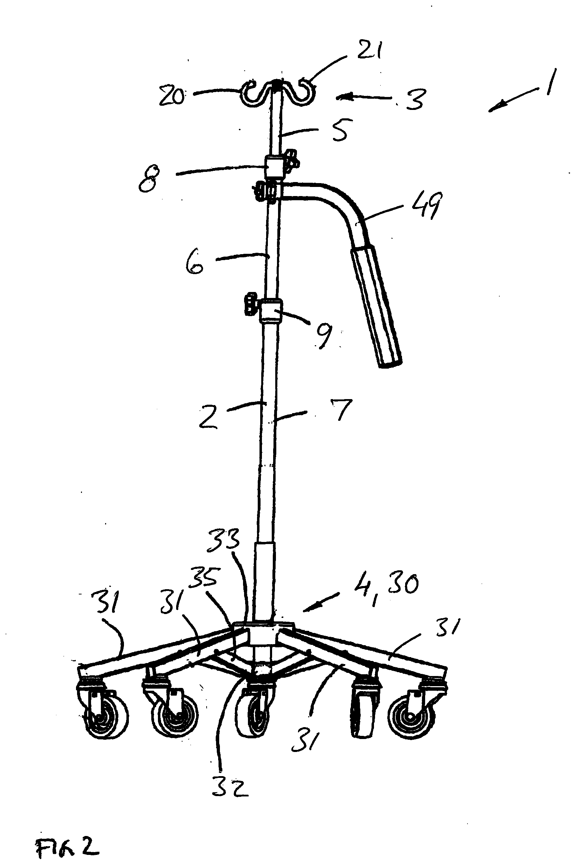

In alternative embodiments of the present invention, it may include at least one handle **49** (shown in figure 2) with which the IV stand may be carried and steered. The handle may be permanently or temporarily attached to the IV stand. Preferably, the handle is temporarily attached to the IV stand's pole 2. The handle may include padding or similar. For example, the handle may include foam or similar material that has been covered with at least one hygienic material layer. The handle allows for the IV stand to be easily and simply carried (brought along) for example in conjunction with home health care or mobile health care.

With reference to figures 6, 7A, 7B, 8A, 8B, 8C and 8D are shown exemplifying variants of systems for suspension of the present invention. The present invention is defined in these embodiments as a system that includes a plurality of IV stands 1 in accordance with one or more embodiments of the invention.

With reference to figure 6, a first embodiment of the present invention is shown where the IV stands are suitable to be suspended in a stand **50.** The stand 50 includes at least one foot (base supporting device) **51,** at least one vertical pole **52** and at least one first suspension device **53** at a first vertical level **54** and at least one second suspension device **55** at a second vertical level **56.** In the exemplifying embodiment, the stand can carry at least eight retracted IV stands per vertical level 54 and 56. In alternative embodiments, it is conceivable that the stand may carry more of fewer retracted IV stands per vertical level than the earlier stated number. The stand's 50 foot 51 is preferably equipped with wheels which results in the stand being able to be moved from one place to another. Further, the stand may be used to move IV stands to and from sterilization or similar. When IV stands are stored in a stand, it reduces the need of floor space to a significant degree.

With reference to figures 7A and 7B, an alternative embodiment of the present invention is shown that may be hung up along a wall or similar. Figure 7A shows how two retracted IV stands 1 may be suspended on a suspension element **57** on a wall or similar. Figure 7B shows how retracted IV stands may be suspended on at least one rod **58** or similar at a first vertical level 54 and preferably also on a second rod **59** or similar on at least one second vertical level 56.

With reference to figure 8A, a variant of a system including a stand in the form of a wagon **60** or similar is shown. The wagon 60 is equipped with wheels. The wagon 60 allows for IV stands to be suspended in at least one first vertical level. The wagon 60 will preferably allow IV stands 1 to be suspended on at least one first vertical level 54 and at least one second vertical level.

The IV stands may for example be moved to and from storage, to and from sterilization and the like with the wheel equipped wagon 60. The need for floor space where the IV stands may be stored is significantly reduced with this design. The wagon is comprised of an arbitrary number of vertical poles **61** (the figure shows four) with a sufficient number of between the vertical poles' intermediate (connecting) parts **62.** The wagon is in the exemplifying embodiment equipped with wheels.

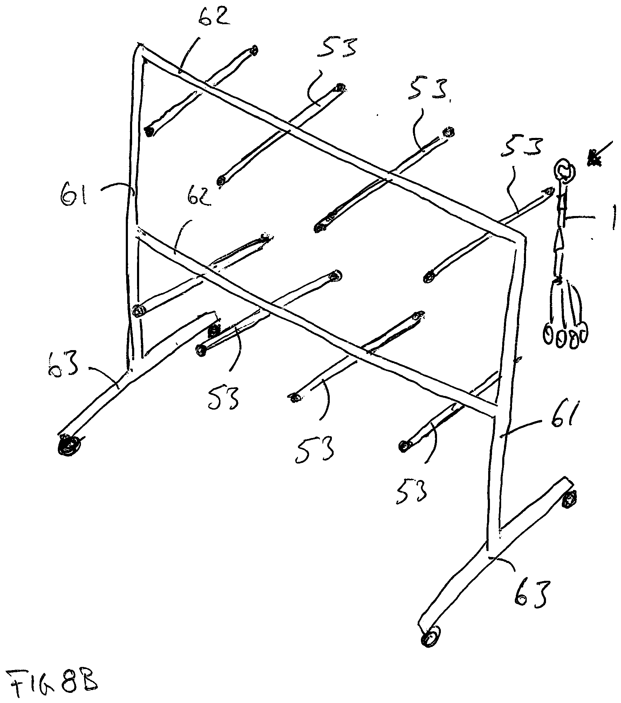

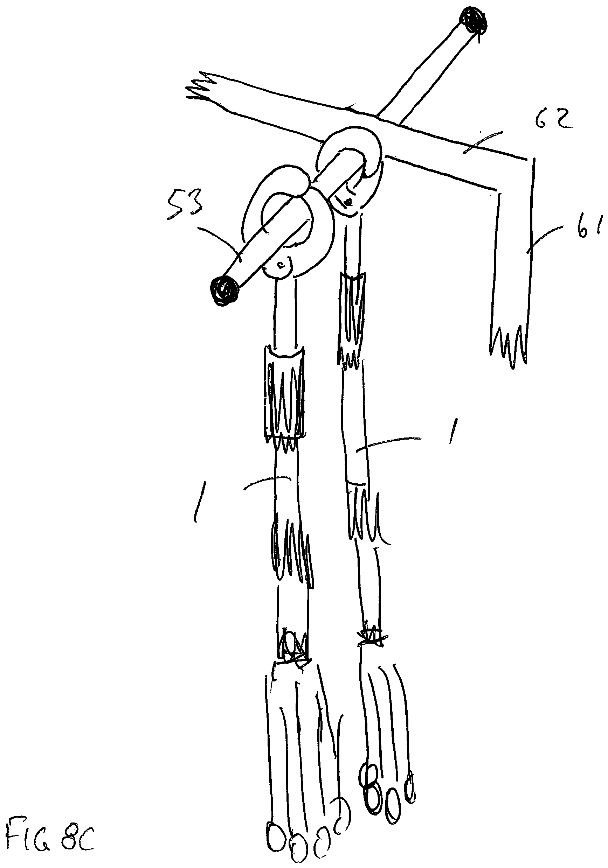

With reference to figures 8B and 8C, an alternative variant of a stand where IV stands are suspended in at least one first vertical level and in at least one second vertical level is shown. The figure also exemplifies how the extended suspension devices 53 allow the IV stands 1 to be hung up when the suspension devices in the IV stand are turned in the upward direction (shown more specifically in figure 8C). In the exemplifying embodiment in figure 8B, the stand includes base supporting devices **63** which are equipped with wheels. It is conceivable that the stand lacks wheels.

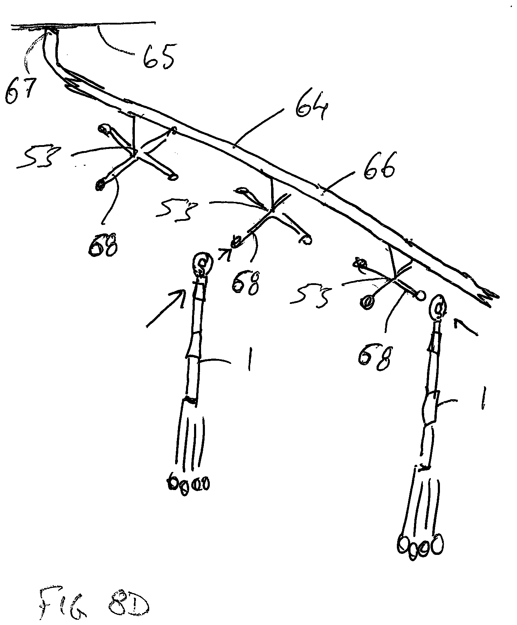

With reference to figure 8D, an alternative system consisting of a ceiling hung suspension device **64** which is attached to the ceiling **65** is shown. The suspension device 64 in the schematically exemplified embodiment is comprised of at least one elongated body **66** such as a rod, a pipe or another elongated body 66. The elongated body 66 is attached to the ceiling with one or more brackets (fasteners) **67.** The brackets 67 may be of some type of previously known brackets. The design includes an arbitrary number of suspension devices 53 with an arbitrary number of suspension members **68.** The suspension members 68 in the suspension devices are preferably elongated (axle, rod or similar) so that the stand may be suspended with the stand's hook-like suspension members turned upward. The stand's turned up suspension members are brought in from the rod's, axle's free end and left hanging until they are to be used. Two or more stands may be hung per rod. At least one other ceiling bracket has been omitted from the figure.

### Advantages of the Invention

The present invention achieves several advantages. Firstly, an improved IV stand and a system for the storage and movement of IV stands is achieved which solves or reduces at least one in the background or in the description mentioned technical problems.

Another advantage is that the stand for IV bags in accordance with the present invention is easily utilized. Another advantage with the IV stand is that it may be retracted so that it takes minimal space to store. A further advantage with the present IV stand is that it includes a suspension device with which the stand may be effectively suspended together with several other stands when it is not used. It is a further advantage with the present IV stand that it is relatively light-weight which thereby allows for easy movement in conjunction with sterilization of the stand and similar. A still further advantage with the IV stand is that it includes fewer parts than previously known stands thereby making it more cost-effective to manufacture and assemble than earlier known types of stands. The IV stand even has the advantage that it is easy to sterilize. The stand is also easy to handle in conjunction with use as an IV stand, in conjunction with sterilization and in conjunction with the stand being moved and stored.

## Claims

1. Stand (1) for IV bags comprised of at least one telescopically arranged pole (2), at least one base supporting device (4) and at least one suspension device (3) on which at least one IV bag may be suspended and that the stand (1) may be suspended when it is not in use, the base supporting device (4) being comprised of one, along sections of the pole's length , turning hub (33) and one fixed hub (32) attached to the pole's lower section and legs (31) whose one end are pivotally attached to the turning hub (33) and the legs via at least one strut (35) are attached to the fixed hub, said strut (35) being pivotally arranged via at least one axle positioned in a transverse recess in the fixed hub, **characterized in**
- **that** the suspension device (3) is comprised of at least one first suspension member (20), at least one second suspension member (21), at least one third suspension member (27) and at least one fourth suspension member (28),
- **that** the pole (2) is comprised of at least one first pole part (5), at least one second pole part (6) and at least one third pole part (7) which are telescopically arranged relative to each other and that the first and second pole parts (5, 6) may be locked together with a t least one first locking device and that the second pole part (6) and third pole part (7) may be mutually locked via at least one second locking device, said locking device being comprised of at least one first locking member and at least one second locking member which via a mutual turning of the connected first and second pole parts (5,6) and second and third pole parts (6,7) in one direction are mutually locked together and with a mutual turning in the other direction are unlocked from one another,
- **that** the fixed hub (32) is comprised of an upper section (36) and a lower section (37) whose upper section (36) and lower section (37) include a corresponding number of grooves (47) as the number of legs (31),
- **that** the turning hub (33) is comprised of a first section (44) and a second section (45) which are connected together with each other via a quick couplers,

2. Stand (1) for IV bags in accordance with claim 1 **characterized in that** the suspension device (3) is comprised of at least one first suspension member (20 and at least one second suspension member (21) which are pivotally attached to the pole (2) around a common axis of rotation.

3. Stand (1) for IV bags in accordance with claim 1 **characterized in that** the suspension device (3) is comprised of at least one first suspension member (20) and at least one second suspension member (21) which are pivotally attached to the pole (2) around separate axes of rotation.

4. Stand (1) for IV bags in accordance with claim 1 **characterized in that** the suspension device (3) is comprised of at least one first suspension member (20) and at least one second suspension member (21) which are pivotally attached to the pole (2) around separate axes of rotation and that the suspension device (3) is comprised of at least one third suspension member (27) and at least one fourth suspension member (28) which are pivotally attached to the pole (2) around separate axes of rotation.

5. Stand (1) for IV bags in accordance with claim 1 **characterized in that** the pole's (2) third part is comprised of at least one internal reinforcement.

6. Stand (1) for IV bags in accordance with claim 1 **characterized in that** the suspension device is comprised of at least one fifth suspension member with which the stand may be suspended in its entirety.

7. Stand (1) for IV bags in accordance with at least one of the previous claims **characterized in that** the suspension members are claw-like.

8. Stand (1) for IV bags in accordance with at least one of the previous claims **characterized in that** the struts and hubs are made of a material wich tolerates temperatures over 100 degrees C.

9. Stand (1) for IV bags in accordance with at least one of the previous claims **characterized in that** the struts and hubs are made of a material wich tolerates temperatures over 140 degrees C.

10. Stand (1) for IV bags in accordance with at least one of the previous claims **characterized in that** the legs' profile is comprise of an open profile.

## Patentansprüche

1. Ständer (1) für IV-Beutel, bestehend aus mindestens einer teleskopartig angeordneten Stange (2), mindestens einer Basisstützvorrichtung (4) und mindestens einer Aufhängevorrichtung (3), an der mindestens ein IV-Beutel aufgehängt werden kann und der Ständer (1) bei Nichtgebrauch aufgehängt werden kann, wobei die Basisstützvorrichtung (4) aus einer entlang von Abschnitten der Stangenlänge drehbaren Drehnabe (33) und einer feste Nabe (32), die am unteren Abschnitt der Stange befestigt ist, und Schenkeln (31) besteht, deren eines Ende schwenkbar an der Drehnabe (33) und die Schenkel über mindestens eine Strebe (35) an der festen Nabe befestigt sind, wobei die Strebe (35) schwenkbar über mindestens eine Achse angeordnet ist, die in einer Queraussparung in der festen Nabe angeordnet ist, **dadurch gekennzeichnet,**
- **dass** die Aufhängevorrichtung (3) aus mindestens einem Aufhängeelement (20), mindestens einem zweiten Aufhängeelement (21), mindestens einem dritten Aufhängeelement (27) und mindestens einem vierten Aufhängeelement (28) besteht,
- **dass** die Stange (2) aus mindestens einem ersten Stangenteil (5), mindestens einem zweiten Stangenteil (6) und mindestens einem dritten Stangenteil (7) besteht, die teleskopartig zueinander angeordnet sind, und dass der erste und zweite Stangenteil (5, 6) mit mindestens einer ersten Verriegelungsvorrichtung verriegelt werden können und dass der zweite Stangenteil (6) und der dritte Stangenteil (7) über mindestens eine zweite Verriegelungsvorrichtung gegenseitig verriegelt werden können, wobei die Verriegelungsvorrichtung aus mindestens einem ersten Verriegelungselement und mindestens einem zweiten Verriegelungselement besteht, die durch eine gegenseitige Drehung der verbundenen ersten und zweiten Stangenteile (5, 6) und zweiten und dritten Stangenteile (6, 7) in einer Richtung gegenseitig miteinander verriegelt sind und durch eine gegenseitige Drehung in der anderen Richtung voneinander entriegelt sind,
- **dass** die feste Nabe (32) aus einem oberen Abschnitt (36) und einem unteren Abschnitt (37) besteht, deren oberer Abschnitt (36) und unterer Abschnitt (37) eine entsprechende Anzahl von Nuten (47) wie die Anzahl der Schenkel (31) aufweisen,
- **dass** die Drehnabe (33) aus einem ersten Abschnitt (44) und einem zweiten Abschnitt (45) besteht, die über eine Schnellkupplung miteinander verbunden sind.

2. Ständer (1) für IV-Beutel nach Anspruch 1, **dadurch gekennzeichnet, dass** die Aufhängevorrichtung (3) aus mindestens einem ersten Aufhängeelement (20) und mindestens einem zweiten Aufhängeelement (21) besteht, die schwenkbar an der Stange (2) um eine gemeinsame Drehachse befestigt sind.

3. Ständer (1) für IV-Beutel nach Anspruch 1, **dadurch gekennzeichnet, dass** die Aufhängevorrichtung (3) aus mindestens einem ersten Aufhängeelement (20) und mindestens einem zweiten Aufhängeelement (21) besteht, die schwenkbar an der Stange (2) um eine separate Drehachse befestigt sind.

4. Ständer (1) für IV-Beutel nach Anspruch 1, **dadurch gekennzeichnet, dass** die Aufhängevorrichtung (3) aus mindestens einem ersten Aufhängeelement (20) und mindestens einem zweiten Aufhängeelement (21) besteht, die um separate Drehachsen schwenkbar an der Stange (2) befestigt sind, und dass die Aufhängevorrichtung (3) aus mindestens einem dritten Aufhängeelement (27) und mindestens einem vierten Aufhängeelement (28) besteht, die um separate Drehachsen schwenkbar an der Stange (2) befestigt sind.

5. Ständer (1) für IV-Beutel nach Anspruch 1, **dadurch gekennzeichnet, dass** der dritte Teil der Stange (2) aus mindestens einer inneren Verstärkung besteht.

6. Ständer (1) für IV-Beutel nach Anspruch 1, **dadurch gekennzeichnet, dass** die Aufhängevorrichtung aus mindestens einem fünften Aufhängeelement besteht, mit dem der Ständer vollständig aufgehängt werden kann.

7. Ständer (1) für IV-Beutel gemäß mindestens einem der vorhergehenden Ansprüche, **dadurch gekennzeichnet, dass** die Aufhängeelemente krallenartig sind.

8. Ständer (1) für IV-Beutel nach mindestens einem der vorhergehenden Ansprüche, **dadurch gekennzeichnet, dass** die Streben und Naben aus einem Material bestehen, das eine Temperatur über 100 Grad C verträgt.

9. Ständer (1) für IV-Beutel nach mindestens einem der vorhergehenden Ansprüche, **dadurch gekennzeichnet, dass** die Streben und Naben aus einem Material bestehen, das eine Temperatur über 140 Grad C verträgt.

10. Ständer (1) für IV-Beutel gemäß mindestens einem der vorhergehenden Ansprüche, **dadurch gekennzeichnet, dass** das Schenkelprofil aus einem offenen Profil besteht.

## Revendications

1. Support (1) pour sacs pour perfusion constitués d'au moins un poteau télescopique (2), au moins un dispositif de support de base (4) au moins un dispositif de suspension (4) sur lequel au moins une poche de perfusion peut être suspendue et le support (1) peut être suspendu lorsqu'il n'est pas utilisé, le dispositif de support de base (4) est constitué, parmi les sections de la longueur du poteau, d'un moyeu mobile (33) et un moyeu fixe (32) attaché sur la section inférieur du poteau et les pieds (31) dont une extrémité est fixée de manière pivotante au moyeu mobile (33) et les pieds via au moins une entretoise (35) sont fixés au moyeu fixe, ladite entretoise (35) étant disposée de manière pivotante par au moins un axe placé dans une cavité transversale dans moyeu fixé **caractérisé par**
- **le fait que** le dispositif de suspension (3) est constitué d'au moins un élément de suspension (20), au moins un deuxième élément de suspension (21), au moins un troisième élément de suspension (27) et au moins un quatrième élément de suspension (28),
- que le poteau (2) est constitué d'au moins une première partie de poteau (5), au moins une deuxième partie de poteau (6) et au moins une troisième partie de poteau (7) qui sont disposées télescopiquement l'une par rapport à l'autre et la première et la deuxième parties du poteau (5, 6) peuvent être verrouillées ensemble avec au moins un premier dispositif de verrouillage et que la deuxième partie du poteau (6) et la troisième partie du poteau (7) peuvent être mutuellement verrouillées par au moins un deuxième dispositif de verrouillage, ledit dispositif de verrouillage étant constitué d'au moins un premier élément de verrouillage et d'au moins un deuxième élément de verrouillage par une rotation mutuelle de la première et deuxième partie du poteau raccordées (5, 6) et la seconde et troisième partie du poteau (6, 7) dans une direction sont verrouillées ensemble mutuellement, par une rotation mutuelle dans l'autre sens elles sont déverrouillées l'une de l'autre,
- Le moyeu fixe (32) est constitué d'une section supérieure (36) et d'une section inférieure (37) dont la section supérieure (36) et la section inférieure (37) inclut le nombre de rainures correspondantes (47) ainsi que de nombre de pieds (31).
- le moyeu mobile (33) est constitué d'une première section (44) et d'une deuxième section (45) qui sont raccordées l'une à l'autre au moyen de raccords rapides.

2. Le support (1) pour sacs de perfusion conformément à la revendication 1 est **caractérisé par le fait que** le dispositif de suspension (3) est constitué d'au moins un premier élément de suspension (20) et d'au moins un deuxième éléments de suspension (21) qui sont fixés de manière pivotante au poteau (2) autour d'un axe de rotation commun.

3. Le support (1) pour sacs de perfusion conformément à la revendication 1 est **caractérisé par le fait que** le dispositif de suspension (3) est constitué d'au moins un premier élément de suspension (20) et d'au moins un deuxième éléments de suspension (21) qui sont fixés de manière pivotante au poteau (2) autour d'un axe de rotation séparé.

4. Le support (1) pour sacs de perfusion conformément à la revendication 1 est **caractérisé par le fait que** le dispositif de suspension (3) est constitué d'au moins un premier élément de suspension (20) et d'au moins un deuxième éléments de suspension (21) qui sont fixés de manière pivotante au poteau (2) autour d'axes de rotation séparés et que le dispositif de suspension (3) est constitué d'au moins un troisième élément de suspension (27) et d'au moins une quatrième élément de suspension (28) qui sont fixés au poteau de manière pivotante (2) autour d'axes de rotation séparés.

5. Le support (1) pour sacs de perfusion conformément à la revendication 1 est **caractérisés par le fait que** la troisième partie du poteau (2) est constituée d'au moins un renforcement interne.

6. Le support (1) pour les sacs de perfusion conformément à la revendication 1 est **caractérisé par le fait que** le dispositif de suspension est constitué d'au moins un cinquième élément de suspension avec lequel le support peut être suspendu dans son intégralité.

7. Le support (1) pour sacs de perfusion conformément avec au moins une des précédentes revendications est **caractérisé par le fait que** les éléments de suspension sont en forme de griffes.

8. Le support (1) pour sacs de perfusion conformément avec au moins une des précédentes revendications est **caractérisé par le fait que** les entretoises et moyeux sont constitués d'un matériau qui tolère des températures de plus de 100 degrés C.

9. Le support (1) pour sacs de perfusion conformément avec au moins une des précédentes revendications est **caractérisé par le fait que** les entretoises et moyeux sont constitués d'un matériau qui tolère des températures de plus de 140 degrés C.

10. Le support (1) pour sacs de perfusion conformément avec au moins une des précédentes revendications est **caractérisé par le fait que** le profil des pieds est constitué d'un profil ouvert.
